# EUROPEAN PATENT APPLICATION

(11) **EP 4 813 358 A1**
(43) Date of publication of application: **30.09.2026**
(21) Application number: 26199106.1
(22) Date of filing: 24.05.2022
(51) Int. Cl.: A61F 7/00, A61F 7/02

(54) **TEAR STIMULATION DEVICE**

(30) Priority: 24.05.2021 GB 202107351
(62) Document number(s) of the earlier application(s) in accordance with Art. 76 EPC: 22732917.4 / 4 346 719
(71) Applicant: University College Dublin, Dublin 4 (IE)
(72) Inventor: CULLEN, Steve, Dublin 4 (IE); BUCKLEY, Sinéad, Dublin 4 (IE); O'CEARBHAILL, Eoin, Dublin 4 (IE); ISELLA, Benedetta, Dublin 4 (IE)
(74) Representative: FRKelly

(57) **Abstract**

The present invention provides a tear stimulation device and method for delivering thermal energy at controlled rates to stimulate the creation of a natural tear by activating the thermo-receptors on the sensory nerves of the eye and extended orbital region, modulation of the thermal energy resulting in controlled, repeated tearing to provide a naturally lubricated and nourishing environment in which the ocular surface can heal.

## Description

### Field of the invention

The present invention provides a tear stimulation device and method, and in particular a device and method which can be utilised in comfort and thus during periods of sleep to ensure the secretion of a regular supply of natural tears in order to treat or avoid dry eye and other conditions associated with or responsible for a lack of natural tears.

### Background of the invention

The cornea (surface of the eye) requires continuous lubrication with tears during both waking hours and periods of sleep in order to function correctly. The tear film and cornea are responsible for 65% of the focusing power of the eye. To maintain optical quality, the tear film must be constantly replenished by natural tear secretion. Without this, the tear film would destabilise, and the ocular surface of the eye would be damaged due to dryness.

The complete tear film is made up of three layers from three different sources:
- An inner mucin layer from the goblet cells
- An aqueous layer from the lacrimal gland
- An outer lipid layer from the meibomian glands

The goblet cells are located in the conjunctiva. The conjunctiva is the tissue that lines the inside of the eyelids and covers the sclera (the white of the eye). The lacrimal glands, one gland for each eye, are located above the eyeball and under the eyebrow. There are also small accessory lacrimal glands in this area, which also contribute to the aqueous layer of the tear. The meibomian glands are located all along the eyelid rims, inside the eyelashes. There are about 40-50 meibomian glands on the upper eyelids and 20-25 glands on the lower eyelids.

It is known that the application of heat to the eyelid can be used to melt lipids in the meibomian gland and improve the secretion from the meibomian glands in particular. This outer lipid layer of the tear prevents evaporation of the inner layers (aqueous, mucin layer) from the surface of the eye, thereby improving dry eye symptoms.

Individuals with dry eye do not, for various reasons, have a sufficient amount of tear lubrication. This can lead to pain, blurred vision, eye infection and anxiety. The conventional daytime treatment of this condition is manual application of eye drops through various mechanisms of action including lubricants, antioxidants, anti-inflammatories, stimulants, steroids and biologics.

Despite all of these daytime treatments, 56% of patients say dry eye disease (DED) symptoms remain the same or become more severe. One reason for this is because DED is not addressed during periods of sleep, most usually at night, and damage to the ocular surface can therefore occur at night where supplementation of tears is not typically possible.

The current treatment options that attempt to address the problem of insufficient lubrication are categorised into tear substitution, tear conservation and tear stimulation. Tear substitution involves manually applied artificially formulated eye drops, ointments or gels. Tear conservation include punctum plugs (invasive implant to block the drainage route from the eye) and moisture goggles, which are worn to create a closed humid environment around the eye. Tear conservation is based primarily on there being enough tear to conserve. Tear stimulation devices seek to stimulate the tear secretion glands by electrical, pharmacological, ultrasonic means either invasively or manually applied externally.

Tear substitution does not use natural tears, are difficult to apply to the eye, have risks associated with the application, and vision is affected during use. Tear conservation relies on the ability to produce a sufficient amount of tears or tear substitution is required as detailed above. Some of the methods are invasive.

Current tear stimulation devices are invasive, usually involving the introduction of a medical device into the body i.e. on the cornea which requires manual application while awake, up the nose which requires manual application while awake, or implantation under the skin which requires a surgical procedure.

It is therefore an object of the present invention to address the above mentioned problems by providing a tear stimulation device and method which is non-invasive, comfortable to wear and thus may be utilised while awake but also during periods of sleep.

### Summary of the invention

According to a first aspect of the present invention there is provided a tear stimulation device comprising a housing; a controller; a power supply; and one or more energy terminals arranged about the housing for the transfer of thermal energy to and from a thermo-responsive region of the face such as to trigger or increase the involuntary activation of tear production; wherein the controller is arranged to cycle the temperature of the one or more energy terminals to deliver sequential heating and cooling phases, and wherein the controller is arranged in at least one of the cooling phases to reduce the temperature of the one or more energy terminals at a rate of between 0.01°C/s and 43 °C/s, more preferably between 3°C/s and 25°C/s, and most preferably between 5°C/s and 20°C/s.

Preferably, the controller is arranged to maintain a fixed temperature at the one or more energy terminals during one or more of the heating and/or cooling phases for a period of between 1 and 6000 seconds, more preferably between 1 and 120 seconds, and most preferably between 1 and 60 seconds.

Preferably, the controller is arranged to set a temperature at the one or more energy terminals of between 0°C and 48°C, more preferably between 5°C and 40°C, and most preferably between 10°C and 35°C.

Preferably, the controller is arranged to modulate the cycle frequency and/or intensity of the thermal energy.

Preferably, the controller is arranged to vary the rate of temperature change during the at least one cooling phase and/or between different cooling phases.

Preferably, the controller is arranged to effect a pulsed temperature change of the one or more energy terminals during at least a part of at least one cooling phase.

Preferably, the controller is arranged to cycle the thermal energy between sequential heating and cooling phases at a frequency of two or more phases per hour, more preferably two or more phases per 10 minutes, and most preferably two or more phases per 3 minutes.

Preferably, the one or more energy terminals comprise a thermoelectric cooler.

Preferably, the one or more energy terminals are positioned to apply the thermal energy to an area on or adjacent one or more lacrimal glands or the supraorbital foramen.

Preferably, the tear stimulation device comprises one or more temperature sensors positioned at or adjacent the one or more energy terminals.

Preferably, the housing comprises a support operable to releasably secure the device to a user or user worn apparel.

Preferably, the support comprises a headband.

Preferably, the one or more energy terminals comprise one or more energy transfer interfaces operable to delivery thermal energy to the thermos-responsive region.

Preferably, the controller is operable to process data from the one or more sensors to implement feedback control of the device.

Preferably, the one or more energy terminals comprise one or more energy transfer interfaces operable to delivery energy to the target area.

Preferably, the one or more energy transfer interfaces are formed integrally with the housing.

Preferably, the one or more energy transfer interfaces are operable to delivery energy to the target area without contacting the target area.

Preferably, the one or more energy transfer interfaces are operable to deliver energy to the target area while in contact with the target area.

Preferably, the one or more energy transfer interfaces comprise one or more contact pads.

Preferably, the one or more contact pads comprise a deformable element.

Preferably, the one or more energy transfer interfaces comprise a heat transfer medium.

According to a second aspect of the present invention there is provided a method of tear stimulation comprising the steps of applying one or more energy terminals to a thermo-responsive region of the face; transferring thermal energy through the one or more energy terminals to and from the thermo-responsive region to sequentially heat and cool the thermo-responsive region; wherein in at least one cooling phase reducing the temperature of the one or more energy terminals at a rate of between 0.01°C/s and 43°C/s, more preferably between 3°C/s and 25°C/s, and most preferably between 5°C/s and 20°C/s.

Preferably, the method comprises maintaining the one or more energy terminals at a fixed temperature during one or more of the heating and/or cooling phases for a period of between 1 and 6000 seconds, more preferably between 1 and 120 seconds, and most preferably between 1 and 60 seconds.

Preferably, the method comprises setting a temperature at the one or more energy terminals of between 0°C and 48°C, more preferably between 5°C and 40°C, and most preferably between 10°C and 35°C.

Preferably, the method comprises modulating the cycle frequency and/or intensity of the thermal energy.

Preferably, the method comprises varying the rate of temperature change during the at least one cooling phase and/or between different cooling phases.

Preferably, the method comprises delivering the thermal energy in pulses during at least a part of at least one cooling phase.

Preferably, the method comprises the step of cycling the thermal energy between sequential heating and cooling phases at a frequency of two or more phases per hour, more preferably two or more phases per 10 minutes, and most preferably two or more phases per 3 minutes.

Preferably, the method comprises applying the thermal energy to an area on or adjacent one or more lacrimal glands or the supraorbital foramen.

Preferably, the method comprises providing the controller with data from one or more temperature sensors positioned at or adjacent the one or more energy terminals.

Preferably, the method comprises electrically and/or physically manipulating the eyelids.

As used herein, the term "energy" is primarily intended to mean thermal energy or changes in temperature, but is also intended to mean mechanical energy such as vibration or massage, acoustic energy, electrical energy which may be modulated in current, voltage, and/or frequency, electromagnetic energy such as gamma rays, X-rays, ultraviolet radiation, visible light, microwaves, radio waves and infrared radiation, chemical energy or a combination of two or more of the above energies.

As used herein, the term "thermo-responsive region" is intended to mean any target area on or about the human head, preferably about the face and most preferably about the orbital region surrounding and including the eyes, hereinafter the extended orbital region, and when energy is applied to this region, for example thermal energy, gives rise to a physiological response in the form of involuntary activation of tear production responsive to the stimulation of cells and/or glands in the region.

As used herein, the term "non-invasive" is intended to mean a non-surgical and potentially noncontact delivery of energy such as thermal energy to a target area of the human head, most preferably the skin of the orbital region of the face, and may for example take the form of the indirect cooling of the target area via an intermediate heat transfer medium.

As used herein, the term "power supply" is intended to mean a local power supply such as a battery or the like and which may be removably connected to the device, or alternatively a means of receiving power from an external source which may be selectively connected to the tear stimulation device, for example by means of a wired or wireless connection.

As used herein, the term "energy terminal" is intended to mean one or more skin contacting regions or elements of a device which, when the device is worn on the head of a user, are in contact with a thermos-responsive region of the face of the user and operable to alter the temperature of said region through thermal conduction, and may for example be defined by a section of a sidewall or surface of a housing of the device, or by one or more discrete elements provided on or about the housing.

### Brief description of the drawings

The present invention will now be described with reference to the accompanying drawings, in which:
Figure 1 illustrates a pictorial representation of the human eye;
Figure 2 illustrates a schematic sectioned side view of the human eye;
Figure 3 illustrates a front elevation of a human head outlining a broad thermo-responsive region;
Figure 4 illustrates a profile view of the human head as illustrated in Figure 3;
Figure 5 illustrates a schematic side elevation of a tear stimulation device according to a first embodiment of the present invention, as applied in use to the human head;
Figure 6 illustrates a front elevation of the arrangement of Figure 5;
Figure 7 illustrates a thermally active region of the tear stimulation device of Figures 5 and 6;
Figure 8 illustrates a schematic side elevation of a tear stimulation device according to a second embodiment of the present invention, as applied in use to the human head;
Figure 9 illustrates a front elevation of the arrangement of Figure 8;
Figure 10 illustrates a thermally active region of the tear stimulation device of Figures 8 and 9;
Figure 11 illustrates a schematic side elevation of a tear stimulation device according to a third embodiment of the present invention, as applied in use to the human head;
Figure 12 illustrates a front elevation of the arrangement of Figure 11;
Figure 13 illustrates a thermally active region of the tear stimulation device of Figures 11 and 12;
Figure 14 illustrates a profile of a human head outlining a specific thermo-responsive region;
Figure 15 illustrates a front view of the human head illustrated in Figure 14;
Figure 16 illustrates a schematic side elevation of a tear stimulation device according to a fourth embodiment of the present invention, as applied in use to the human head;
Figure 17 illustrates a front elevation of the arrangement of Figure 16;
Figure 18 illustrates a thermally active region of the tear stimulation device of Figures 16 and 17;
Figure 19 illustrates a profile of a human head outlining a specific thermo-responsive region;
Figure 20 illustrates a front view of the human head illustrated in Figure 19;
Figure 21 illustrates a schematic side elevation of a tear stimulation device according to a fifth embodiment of the present invention, as applied in use to the human head;
Figure 22 illustrates a front elevation of the arrangement of Figure 21;
Figure 23 illustrates a thermally active region of the tear stimulation device of Figures 21 and 22;
Figure 24 illustrates a font elevation of the tear stimulation device of Figures 21 and 22 in isolation;
Figure 25 illustrates a profile of a human head with eyes open and outlining a specific thermo-responsive region;
Figure 26 illustrates a front view of the human head illustrated in Figure 25;
Figure 27 illustrates the profile of Figure 25 with eyes closed;
Figure 28 illustrates the front view of Figure 26 with eyes closed;
Figure 29 illustrates a schematic side elevation of a tear stimulation device according to a sixth embodiment of the present invention, as applied in use to the human head;
Figure 30 illustrates a front elevation of the arrangement of Figure 29;
Figure 31 illustrates a thermally active region of the tear stimulation device of Figures 29 and 30;
Figure 32 illustrates a schematic side elevation of a tear stimulation device according to a seventh embodiment of the present invention, as applied in use to the human head;
Figure 33 illustrates a front elevation of the arrangement of Figure 32;
Figure 34 illustrates a thermally active region of the tear stimulation device of Figures 32 and 33;
Figure 35 illustrates a profile of a human head outlining a specific thermo-responsive region;
Figure 36 illustrates a front view of the human head illustrated in Figure 35;
Figure 37 illustrates a schematic side elevation of a tear stimulation device according to a eight embodiment of the present invention, as applied in use to the human head;
Figure 38 illustrates a front elevation of the arrangement of Figure 37;
Figure 39 illustrates a thermally active region of the tear stimulation device of Figures 37 and 38;
Figure 40 illustrates a font elevation of the tear stimulation device of Figures 37 and 38 in isolation;
Figure 41 illustrates a profile of a human head outlining a specific thermo-responsive region;
Figure 42 illustrates a front view of the human head illustrated in Figure 41;
Figure 43 illustrates a schematic side elevation of a tear stimulation device according to a ninth embodiment of the present invention, as applied in use to the human head;
Figure 44 illustrates a front elevation of the arrangement of Figure 43;
Figure 45 illustrates a thermally active region of the tear stimulation device of Figures 43 and 44; and
Figure 46 illustrates a schematic representation of the working components of a tear stimulation device according to the present invention.

### Detailed description of the drawings

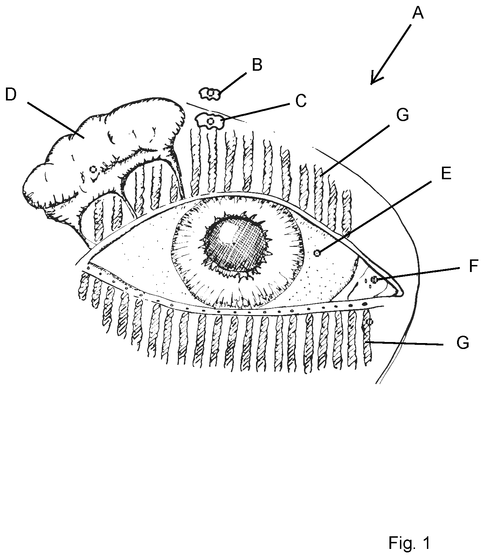

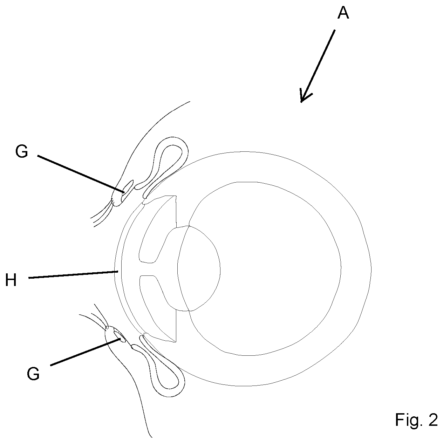

Referring now to the accompanying drawings, Figures 1 and 2 illustrate component parts of the human eye A, noting a number of cells and glands which can define part of a thermoreceptor pathway involved in the involuntary production of tears as discussed above. Figure 1 illustrates the Kraus Gland B, Wolfring Gland C, Lacrimal Gland D, Goblet Cells E, Popov Glands F, and Meibomian Gland G. Figure 2 illustrates the cornea H and the Meibomian Glands G.

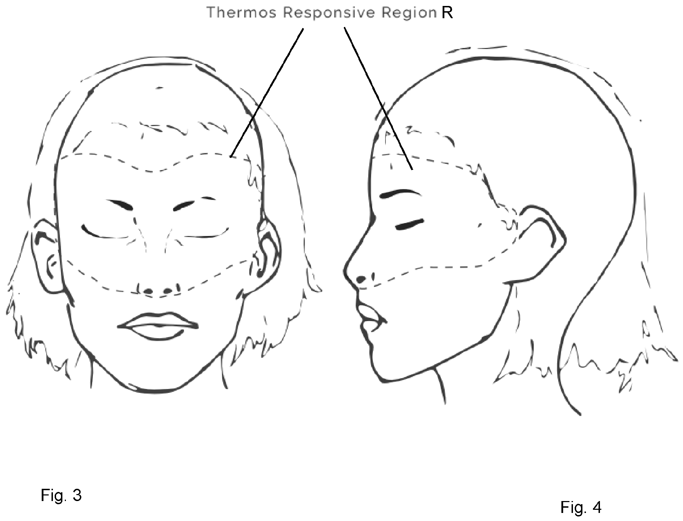

As described hereinafter in detail it has been found that the controlled application of thermal energy to a thermo-responsive region R of the human head, shown schematically in Figures 3 and 4, can be utilised to effect or increase the involuntary production of tears in order to provide the necessary lubrication to the eye. This has particular application to persons suffering from various conditions which result in the reduction of absence of tear generation, in particular during periods of sleep, and can thus be used to treat such conditions. The type and format of the thermal energy delivered to the thermo-responsive region R may vary, for example delivered over a period of time and/or modulated in frequency of application, intensity, period of application, cycles of heating and cooling, rate of change of temperature, etc. and may be delivered to one or more specific sites within the region R, whether simultaneously, sequentially, or otherwise and as set out in greater detail hereinafter. The thermal energy may be delivered while the user is awake or asleep, with eyes open or closed, but the invention is particularly intended to allow energy delivery during periods of sleep when the eyes are closed.

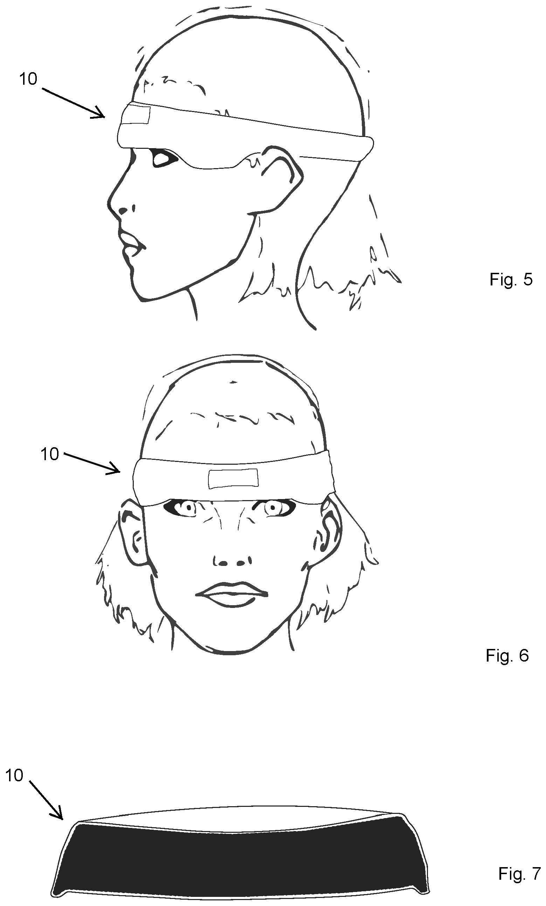

Referring now to Figures 5 to 7 there is illustrated a first embodiment of a tear stimulation device according to the present invention, generally indicated as 10, and which is provided in the form of a headband which is preferably at least partially formed from a stretchable or elastic and soft material such as to be self-retaining and comfortable to wear. This allows the device 10 to be worn during periods of sleep, despite the user being illustrated with eyes opened. The device 10 may also be held to the desired location by other means e.g. microneedles, adhesive, suction pads, Velcro, magnets, etc. The device 10 is preferably adjustable to different head sizes i.e. being stretchy, malleable or adjustable in size via any suitable means (not shown). The device 10 may use the ears, nose, hairline, skin texture or bone structures to be held in place. The device 10 of the exemplary embodiment shown in Figures 5 and 6 is shaped and dimensioned to overlie the brow line adjacent the orbital region but could of course be of any other suitable shape, as detailed by the further embodiments described hereinafter.

The device 10 is arranged to deliver thermal energy to a target region lying within the thermo-responsive region R shown in Figures 3 and 4, and in particular is arranged to heat and cool the target region about the brow line. The device 10 is preferably but not exclusively arranged to apply thermal energy at the supraorbital foramen towards the ear such as to cover the channels from the lacrimal glands (starting at the supraorbital i.e. the medial of the iris when eye is looking straight ahead and stretching 30mm). In particular the device 10 is arranged to overlie the lacrimal gland (position shown in Figure 1) above at least one and preferably both eyes, in order to apply thermal energy to the area of skin overlying and/or adjacent the lacrimal gland, which it has been found is the most effect thermo-responsive region in stimulating tear production. The active cooling region or energy terminal(s) of the device 10 is illustrated in Figure 7. It will be appreciated that this is an exemplary heating and cooling region, which could be of any other suitable form, for example a plurality of discrete hearting and/or cooling regions. Additional and/or alternative target areas are disclosed in the subsequent embodiments described hereinafter. It is also to be understood that this exemplary embodiment utilises thermal energy in the form of sequential heating and cooling as the sole form of energy delivered to the target area, but one or more additional forms of energy could be delivered. Thus reference to heating, cooling or thermal energy should be understood to encompass such.

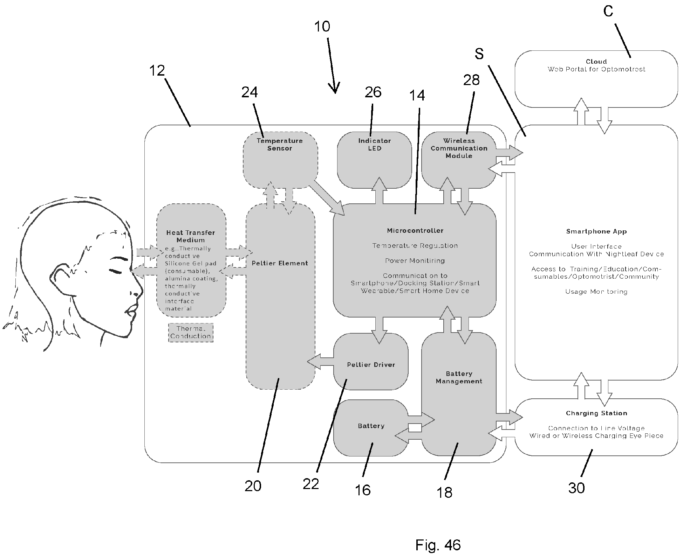

Figure 46 illustrates a schematic representation of one exemplary combination of hardware components which the device 10 may comprise, but it will be understood from the following description of the operation of the device 10 that this is a non-limiting example and there are many alternative components (not shown) and combinations that could be utilised to achieve the required operational functionality. In the embodiment illustrated all of the components illustrated schematically in Figure 46 are fully contained with the headband of the device 10 but it is also envisaged that one or more components, for example a power supply in the form of a battery, may be externally located and/or releasably attached to the device 10. Furthermore, while the stimulation device 10 is shown as a dedicated or fully contained device 10, it is also envisaged that the stimulation device 10 of the invention could be provided in other form factors, for example to be fitted in and/or supported on other items worn by a user, for example a pair of glasses, a wig, jewellery, hat, face mask or any other item worn on or about the head.

The device 10 of this exemplary embodiment comprises a housing 12 containing a controller 14 on which a control algorithm is programmed to provide the ability for autonomous operation of the device 10. The device 10 further comprises a power supply in the form of a battery 16 and battery management module 18 supplying the controller 14 with power. It is envisaged that the battery 16 could be replaced or augmented with energy harvested from the used, whether thermal, kinetic or otherwise. The controller 14 operates at least one energy terminal comprising at least one peltier element 20 and preferably an energy transfer interface 22 which may be provided in various forms as hereinafter described. In practice the device 10 will preferably include at least a pair of the peltier elements 20 in order to stimulate the thermo-responsive region above each eye. A peltier driver 22 is included to effect operation of the or each peltier element 20. The energy transfer interface 22 may for example comprise or be located adjacent a skin contacting surface of the headband, in order to facilitate heating and cooling of the thermo-responsive region. The energy transfer interface 22 may comprise one or more energy transfer mediums such as a solid, a liquid such as a gel, and/or a gas such as air, etc. in order to achieve a controlled and targeted delivery of thermal energy from the peltier element 20. A gaseous interface may result in enhanced activation of the mechanoreceptors, thermoreceptors and other nerves in the skin of the target area, as these are more sensitive when there is no solid/liquid interface presence on the skin. The controller 14 is operable to effect the generation and application of energy at controlled rates and/or physical displacement to create a natural tear by activating appropriate receptors on the sensory nerves of the eye and orbital region. The modulation of this energy results in a controlled, repeated tearing. This provides a naturally lubricated and nourishing environment in which the ocular surface can heal, in particular during periods of sleep, and thus generally overnight.

The controller 14 is therefore preferably adapted to autonomously operate the various components, in particular the peltier element(s) 20, to allow the device 10 to be used during periods of use, in particular sleep, without requiring any user input. The algorithm running on the controller 14 is thus operable to utilise relevant data as inputs and provide appropriate control outputs to the respective components of the device 10 in order to effect the desired operation thereof. The algorithm is operable to incorporate feedback control to allow the operation of device 10 to be adapted to various external parameters as detailed hereinafter, in particular to provide temperature feedback control. It is of course to be understood that user control of the device 10 is possible, and may for example be achieved through a smartphone S or the like, either locally via a wired or wireless connection such as Bluetooth^{™}, near field communication (NFC) or the like, or remotely via the cloud C. This connectivity can also enable a medical profession or the like to remotely access or monitor data on the operation of the device 10 and the condition of the user, allowing the medical professional to monitor the patient and/or modify the treatment programme based on said feedback.

The heat transfer medium associated with the energy transfer interface 22 defining the thermally active area of the device 10 may be contained within a suitable enclosure (not shown) such as a fluid impermeable reservoir which may for example be captured between layers of material forming the headband. It will of course be appreciated that the energy terminal may comprise alternative or additional means of energy generation and delivery to the peltier element 20, which may for example be operable to deliver mechanical energy, electromagnetic energy, chemical energy, etc. and in each case the energy transfer interface 22 is appropriately selected, or may in certain cases be omitted. The energy transfer interface 22 could for example be in the form of an optical wave guide (not shown) to direct light onto the target area. The energy transfer interface 22 may be arranged to be in direct contact with the target area, or out of contact and operable to delivery energy onto the target area, for example over a relatively short distance. It is however preferable to locate the peltier element 20 to be as close to the skin of the thermo-responsive area when the device 10 is applied to the user's face or head. In an exemplary arrangement the skin contact material of the device 10 overlying the peltier element 20 will have a thermal conductivity no less than 429 W/mK and at a thickness no greater than 1mm, and should preferably closely conform to the adjacent surface of the peltier cell(s) 20 to maximise heat transfer, while also preferably match or complementing the shape of the housing 12. Examples of suitable materials are silver and alumina but any other alternative may be employed, which should also be biocompatible, in particular to orbital skin.

The device 10 further comprises a temperature sensor 24 which is operable to provide information to the algorithm running on the controller 14 regarding the temperature of the Peltier element 20 and/or the energy transfer interface 22, and/or the ambient temperature of the environment or the skin or the user. The temperature sensor 24 is preferably arranged to monitor the temperature of both a "hot" and a "cold" side of the Peltier element 20, and may for example comprise two dedicated temperature sensors for this purpose. The temperature sensor 24 is preferably located as close as possible to the Peltier element 20 in order to reduce undershoot and/or overshoot of the required temperature to be applied by the Peltier element 20. An optional indicator such as an LED 26 allows status signals or other basic information regarding the device 10 to be provided to a user. The device 10 preferably also comprises a communication module 28 which is preferably capable of wireless communication in order to allow the device 10 to be connected and preferably controller from an external interface, for example via a smartphone S or the like. A charging station 30 is also shown, which does not form part of the device 10 but with which the device 10 may be interfaced to allow recharging of the battery 16 in known fashion. Charging may be achieved wirelessly in order to avoid the requirement for an external power socket on the device 10.

The device 10 may additionally comprise one or more sensors (not shown) operable to provide information on one or more physical and/or environmental conditions such local body temperature, tear gland activity, heart rate, hormone levels, air temperature and/or humidity, motion, orientation, sleep cycle, one or more external information sources, etc. and which sensors can communicate with the controller 14 in order to allow autonomous feedback control of the device 10. For example the device 10 may comprise one or more heat sinks to dissipate thermal energy generated, for example from the Peltier element 20, and the device 10 may be operable to direct this thermal energy to the most appropriate heat sink, potentially depending on the orientation of the device 10, which will be determined by the position of the user's head. For example if a user is lying with one side of their head against a pillow or the like, it may not be appropriate or effective to utilise a heat sink on that side of the device 10, and so the controller 14 may be operable to select a heat sink on the opposite exposed side of the device 10 from which heat can more readily escape. Additional decision making functionality may of course be provided and controlled by the algorithm running on the controller 14.

The tear stimulation device 10 of the invention may incorporate one or more systems (not shown) for holding the eyelid closed, in particular to prevent evaporation of the newly stimulated tears, and furthermore to protect from contact or other irritants, to reduce light incidence, and/or to maintain a consistent temperature about the eye. This will also be beneficial for the treatment of nocturnal lagophthalmos, where dysfunctional eyelids mean they do not close fully during periods of sleep.

The device 10 may also include one or more systems (not shown) to massage the eyelid to prevent it from sticking to the eye (cornea) along the inner surface of the eyelid in the case of poor lubrication between the surfaces. The device 10 may additionally include one or more systems (not shown) to maintain the eyelid partially open in order to allow the energy modulation access to the cornea, which may for example take the form of electrical stimulation to the eye region.

The device 10 may for example be adapted to manipulate the eyelid by either mechanical means or electrical means to prevent the eyelid sticking when lack of lubrication is an issue, to facilitate improved energy transfer to the cornea/eyeball surface. Eyelid manipulation can also be employed to move the freshly stimulated tears across the eye surface or to keep the eyelid closed to reduce evaporation of the tears stimulated. Mechanical means of moving the eyelid could for example comprise a material in contact with the external eyelid and moved by suitable mechanical and/or electrical means (not shown), for example one or more servos, piezoelectric actuators, etc.. Electrical means of manipulating the eyelid could also be achieved by electrical stimulation of the nerves responsible for the contraction and relaxation of the eyelid muscles.

In use a user applies the device 10 as illustrated in Figures 5 and 6 such that the active heating and cooling region of the device 10 as illustrated in Figure 7 is correctly located. The device 10 may be manually activated, or may utilise data from one or more sensors (not shown) to detect that the user is wearing the device 10 and/or sleeping and thereby trigger activation of the device 10. Alternatively the device 10 may be programmed to operate during pre-set times of the day or for a pre-set period of time. Once operational the device 10 provides non-invasive delivery of thermal energy to the target area, and the controller 14 is operable to modulate said energy such as to trigger the involuntary activation of tear production. The energy modulation may affect tear secretion by impacting the neural pathway for tear secretion in different places e.g. thermoreceptors, mechanoreceptors, polymodal receptors, the nerves or on the glands. These receptors are located in various regions of the target area, and the device 10, and in particular the thermally active region as shown in Figure 7, may be configured to apply energy to specific receptors or groups of receptors. For example the device 10 may target receptors in the skin of the orbital region, most notably over or about the lacrimal gland, the skin of the eyelid, skin and inner mucosa of the nose, and the cornea through open or closed eyelids. Target areas may therefore be the eyebrow, forehead, nasal area, orbital region, temple adjacent the orbital region, the outside and/or inside of the nose, and the cheekbones or any combination of the above. The device 10 is preferably operable to generate sequential thermal energy cycles over a specified time period as detailed below. The application of additional forms of energy, whether constant or modulated may assist in affecting tear secretion by impacting the neural pathway for tear secretion in different places e.g. thermoreceptors, mechanoreceptors, polymodal receptors, the nerves themselves or on the glands.

The device 10, and in particular the controller 14, is operable to deliver thermal energy to the target area, and during use to modulate or vary the energy profile in order to stimulate the requisite cells and/or glands to affect tear secretion. For example the controller 14 may be programmed to modulate the temperature of the Peltier element 20 in order to modulate the temperature at the thermo-responsive target site. The modulation parameter will however vary depending, for example, on the forms of energy being applied, physiological conditions, sleep state, etc. and can be autonomously modified based on feedback from one or more sensors (not shown). If mechanical energy such as massage is being applied in combination with thermal energy, the frequency and intensity of the massaging may be modulated. If electromagnetic energy is being applied, the frequency, wavelength and/or intensity may be varied. The length of time that the energy is applied may also be modulated, as may be the length of intervening periods during which no energy is applied.

The device 10 may operate a hierarchical control scheme, including at an upper level a programme which covers an entire period of use, for example an overnight or sleep programme. A programme is therefore the complete period of time that the device 10 is intended to be used in one treatment session. All programmes are relevant for waking hours in addition to periods of sleep. For example the device 10 may be used while asleep or awake purely to produce tear secretion, hereinafter referred to as operating a tear secretion programme, but equally may be used to facilitate sleep, hereinafter referred to as operating a sleep facilitation programme. The sleep facilitation programme may operate with or without tear secretion, for example to simply relax the wearer through the application of heat, massage, etc. without stimulating tear secretion, or in advance of stimulating tear secretion. It is however envisaged that the primarily use application of the device 10 will be to solely establish tear secretion at night or during periods of sleep. Each programme includes a number of phases, while each phase may define a number of "unit operations". Each unit of operation may comprise a number of individual cycles as set out hereinafter. The algorithm running on the controller 14 is programmed to implement the appropriate control scheme for the respective programme and to incorporate feedback control based on data received from the one or more sensors or other sources.

At the lowest tier of the hierarchical control scheme is an array of different thermal energy cycles that may be implemented by the device 10. The different defined energy levels and rates of change of energy within a cycle can be influenced by factors such as resting body energy or energy input from the device 10. An energy cycle may consists of bringing the thermal energy to a starting defined level at a defined rate, then changing the energy at a defined rate, holding for a defined period of time at the new energy level, and changing at a defined rate to an ending defined energy level. The rates of changes of energy may be of various profiles, for example but not limited to a sine wave, linear and stepwise profile. An energy cycle may also comprise a hold cycle which may consists of the sources of energy controlled by a cycle being held at a defined energy level for a defined period of time, and/or the sources of energy may also be controlled by a cycle to be turned off for a defined period of time.

In particular it has surprisingly been found that the cyclic application of thermal energy in sequential heating and cooling phases to the thermo-responsive region is particularly effective in stimulating tear production, and most notably it has been found that an elevated rate of temperature change during the cooling phase has a significant and surprising impact on tear stimulation. In particular it has been discovered that a rate of change of temperature during the cooling phase of between 0.01°C/s and 43°C/s, more preferably between 3°C/s and 25°C/s, and most preferably between 5°C/s and 20°C/s at the thermos-responsive target site results in significant tear generation. This rapid cooling of the thermo-receptor stimulates the thermo-receptors to a surprising level, in order to effect significant complete tear stimulation. Once the cooling phase is complete the device 10, under the action of the controller 14, utilises the peltier cell(s) 20 in order to heat the thermo-responsive area. This has the effect of returning the stimulated thermo-receptors to a precise baseline temperature at a specific rate in order to reset the sensitivity of the thermo-receptors, in particular with respect to the immediately following cooling phase implemented by the device 10. In addition to bringing the thermo-receptors back to this baseline temperature, the heating phase can act to clear blocked Meibomian glands by heating trapped meibum secretion and allowing it to flow to clear the gland, thereby further improving overall tear quality. Furthermore, the elevated rate of temperature change during the cooling phase additionally results in activating the so called "blink response" which augments the action of the device 10 in achieving tear stimulation, both by evenly coating the cornea with tear, and by cooling the cornea by direct contact with the eyelid which has been rapidly cooled by the device 10. In one study, the blink rate more than doubled from an average of twenty blinks per minute to an average of forty five blinks per minute when the device 10 was applied, using a cooling rate of 5°C/s, as measured using video analysis.

By precisely controlling the rate of temperature change and absolute temperature applied the thermo-receptors are given regular input to effectively retrain the thermo-receptor response to the ambient temperature experienced during normal biological function. The device 10 is thus operable to repair or retrain damaged thermo-receptors.

The efficacy of the tear stimulation device 10 was appraised using a Myah^{™} as supplied by Topcon Healthcare, an ocular testing and screening apparatus used in the field of Optometry practice to provide data regarding a number of indicators of tear stimulation, in particular Tear Meniscus Height (TMH).

Testing using the Myah^{™} employed the following test protocol:
A. The baseline reading of the Tear Meniscus Height (TMH) is read by the subject, in a seated position, placing their chin in the Myah^{™} chin hold, the forehead against the Myah forehead strap and looking straight into the Myah^{™} device.
B. The operator of the Myah^{™} selects the "TMH" measurement option from the on screen menu. The operator then adjusts the Myah^{™}, while the subject remains still, to focus the Myah^{™} cross hairs onto the tear meniscus (lower meniscus is measured at the bottom of the eye).
C. The focussing on the TMH of the eye under study is done by fine movement of the Myah^{™} joystick.
D. Once the operator has focussed on the TMH, the button on the Myah^{™} joystick is pressed and an image is taken of the TMH.
E. The image is then analysed using the Myah^{™} interface. The image is optimised by the Myah^{™} software to accentuate the tear meniscus. The upper and lower edge of the TMH is identified on the magnified image and the Myah^{™} calculates the TMH.
F. Without moving from the seated position, the subject then removes their head from the Myah^{™} and the device 10 of the invention is placed on the thermos-responsive area of the orbital region and a predefined temperature algorithm is executed by the device 10.
G. Once the predefined temperature algorithm has been completed, the subject removes the device 10 and the TMH is measured again as per the steps A to E above.

An exemplary thermal energy cycle of the device 10 tested using the Myah^{™} involved bringing the thermally active area of the device 10, namely that region in direct thermal communication with the peltier cell(s) 20, from 37°C, to 20°C at a defined rate of change 6°C/s. Across two tests this rate of temperature change in the cooling phase gave an increase in TMH of 0.21mm and 0.15mm. Another example of a temperature cycle or sequence effected by the device 10 and evaluated using the Myah^{™} involved a temperature drop from 35°C to 25°C at a rate of change of 4°C/s for 2.5 seconds, then from 25°C to 23°C at a rate of change of 0.2°C/s for 10 seconds and then raising the temperature up to 35°C at a rate of 2°C/s, and repeating this cycle twice more. This gave an increase in TMH of 0.16 mm.

A further sequence tested involved a temperature drop from 35°C to 26°C at a rate of change of 6°C/s for 1.5 seconds, then from 26°C to 23°C at a rate of change of 0.3°C/s for 9 seconds, then holding at 23°C for 18 seconds before a heating phase with heating up to 35°C at a rate of 2.5°C/s for 2 seconds to give an increase in TMH across three tests of 0.12mm, 0.18mm and 0.19 mm.

Another tested sequence employed a temperature drop from 35°C to 25°C at a rate of change of 2°C/s for 5 seconds, then from 25°C to 20°C at a rate of change of 1°C/s for 5 seconds and then back up to 35°C at a rate of 1°C/s heating for 5 seconds, repeating this cycle nine more times to give an increase in THM of 0.25 mm.

The above tests were carried out in locations with environmental temperature ranging from 16°C to 19°C and the device 10 placed on the skin of participants under test in order to achieve the stated temperature changes at the specified rates.

It will be appreciated that the above tests are exemplary and a large number of alternative cycles, sequences, temperatures and rate changes may be employed to achieve desired outcomes or to treat particular cases. For example to control the tear secretion for a reflex tear and then a basal tear the sequence may involve a heating phase with heating to 35°C at a rate of 1°C/s and holding for 120 seconds. Then a cooling phase with cooling to 10°C at a rate of 25°C/s and holding for 20 seconds. Then a further heating phase with heating to 35°C at a rate of 2°C/s, holding for 60 seconds. Then a cooling phase with cooling to 20°C at a rate of 15°C/s and hold for 20 seconds. Then heating to 35°C at a rate of 2°C/s, holding for 60 seconds. Then cool to 20°C at a rate of 15°C/s and hold for 20 seconds.

At the next tier up in the control scheme are stages, and the programme for tear secretion and the programme for sleep facilitation may consist of different stages, for example a first stage for relaxation during which period a user has time to physically and/or mentally relax. A second or pre-sleep stage, immediately before the period of time intended for sleep. A third or sleep stage defining the period of time intended to be dedicated to sleep, and a fourth or post sleep stage immediately after waking.

The stages are comprised of one or more of the unit operations. Each stage can have multiple unit operations, in any order. The unit operations for tear secretion may be different to the unit operations for sleep facilitation. The unit operations for tear secretion may be continuous such as to mimic a blink, on multiple occasions. The unit operations for tear secretion during sleep facilitation may be continuous such as to mimic a closed eye tear, being defined as the lubrication of the eye during prolonged eye closure particularly at night when sleeping but can also be while awake. The unit operations may define a flush to mimic a yawn to produce a concentrated, exaggerated and continuous tear, which may be achieved through a more frequent and intense energy fluctuation. A further unit operation may be defined as a maintenance unit operation in order to maintain the consistency of the meibum in the meibomian glands of the eye and the consistency of all the oil in all the glands of the skin which could be affected by the reduction of temperature of the previous two or any "unit operations". A unit operation is a pattern formed by one or multiple simultaneous cycles, at different locations in the target zone or by energy sources at different frequency intervals. Examples are an energy wave or gradient pattern across the thermally active area of the device 10 and for example extending horizontally, vertically, and/or diagonally, concentric circles, multiple alternating pulses, etc. generated on the thermally active area of the device 10.

As noted above the device 10 may have sensors (not shown) which may be used to measure markers for sleep, eye, brain activity, REM, sleep patterns, etc. This data may be used to inform the programmes for the tear secretion and for facilitating sleep/relaxation. The data may be used to improve understanding of dry eye and sleep e.g. climate aspects, quantity of exercise, body hydration, medication usage, food/supplements, other influences such as contact lens, hormonal, reading time, driving time, screen time, recording of signs and symptoms. This information may be provided to a medical professional or the like to allow appropriate review.

The number of cycles and the other variables listed above may be controlled by feedback from the sensors (not shown) or other sources or data points (e.g. based on temperature, tear production, wetness or other change in indicator, daily activities, computer usage, exercise, hours slept the night before, environmental conditions of sleep or of the day). The device 10 may operate in a closed loop or semi-closed loop mode, operable to detect directly or indirectly the level of tear production and adjust the control system in real-time or on a follow-up basis.

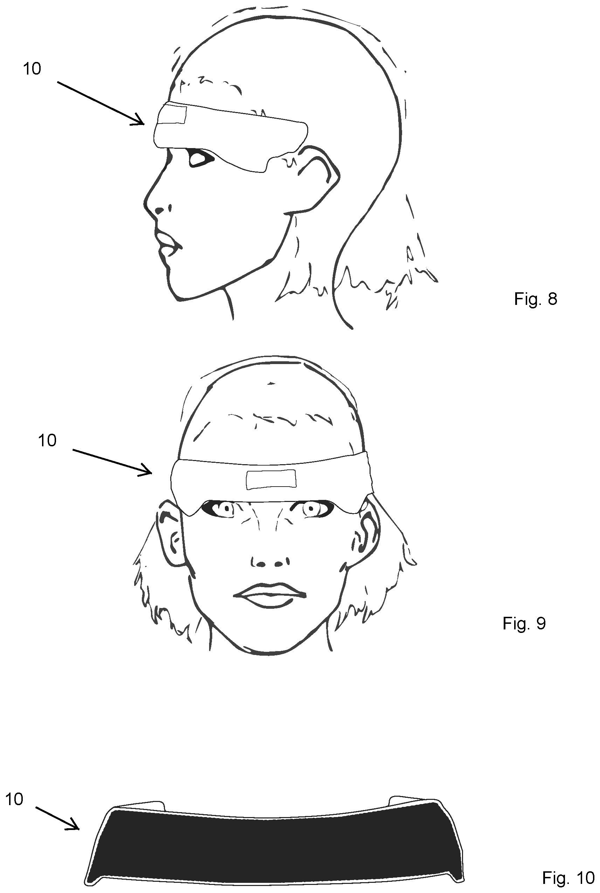

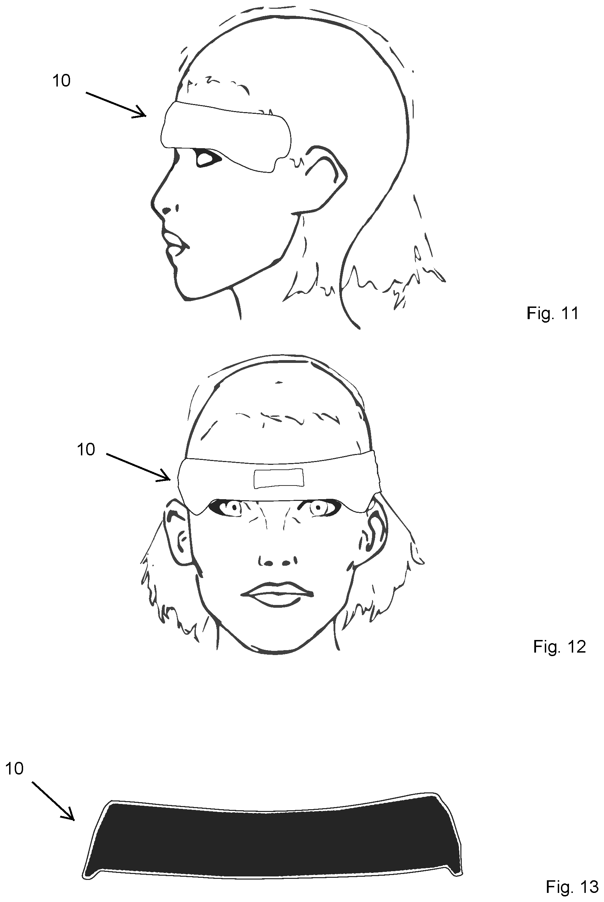

The device 10 illustrated in Figures 5 to 13 was tested on a sample group of users to demonstrate efficacy, and using an exemplary control scheme. The eyes were closed for the test. A setpoint of 20°C was applied at the energy terminals for a period of twenty seconds. The tear menisci serve as reservoirs, supplying tears to the pre-corneal tear film. The majority of tear fluid is contained within the menisci, formed by the tears lying at the junctions of the bulbar conjunctiva and the margins of both the upper and lower eyelids. A tear meniscus height of less than 0.25mm is suggestive of dry eye. The test results showed a 68% increase in tear meniscus height following use of the device 10.

The tear stimulation device according to the invention may be provided in a large number of form factors, which can be designed for example to suit particular applications, user preferences, control schemes, target areas to which thermal and optionally other energy is to be delivered, along with various other factors. Figures 5 to 13 illustrate examples of the device 10 which are variations of the headband form factor and are designed to apply thermal energy to a thermo-responsive target area along the brow adjacent the orbital region, and may be used during periods of sleep or while a user is awake as the device 10 does not cover the eyes.

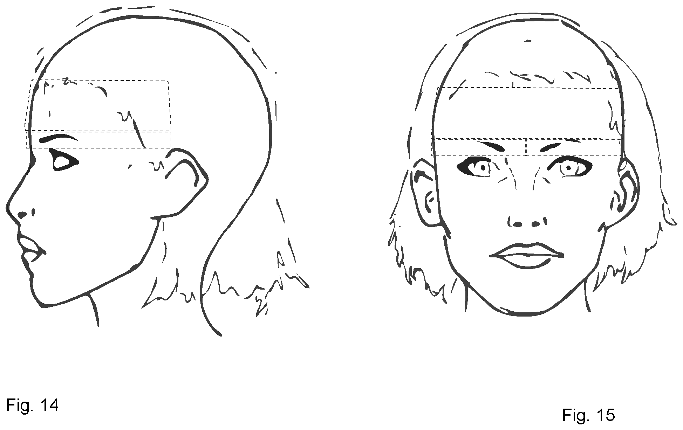

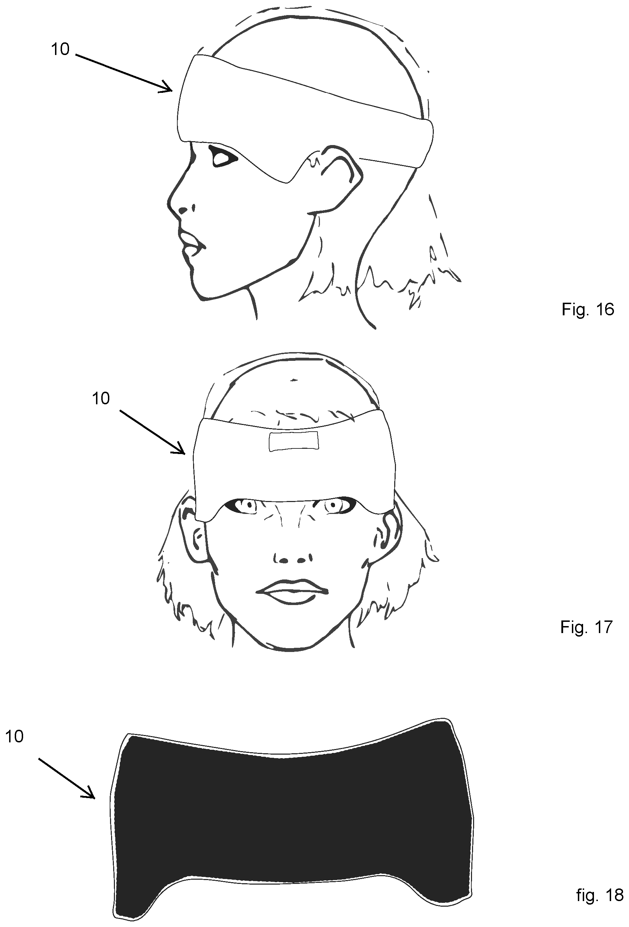

Figures 14 and 15 illustrate an enlarged target area including the brow and the forehead, while Figures 16 to 18 schematically illustrate an embodiment of the device 10 designed to delivery thermal energy to this target area, with Figure 18 highlighting the thermally active area of the device 10. It should be understood that the thermally active area may also contain mechanical or other forms of energy delivery, and the term "thermally active area" should be construed throughout to potentially encompass these alternative forms of energy. As with the previous embodiments this version of the device 10 may be used during periods of sleep and while a user is awake.

An exemplary control scheme for these devices 10 may involve bringing the localized temperature, through cooling, to between 0.01 and 43 °C, more preferably from 37°C to 20°C for comfort and tolerability in the orbital region. The device 10 may comprise a thermal energy interface in the form of a heat transfer medium of silicone and/or air defining the thermally active area. A temperature drop from 37°C to 20°C at a rate of change of 6 °C/s provides the required temperature drop to activate the cold thermoreceptors in the skin of the eyelid. The amount of temperature drop may vary (to avoid the thermoreceptors becoming used to the stimulus and therefore not responding). The duration of a cycle may vary in length, as may the frequency of cycles, for example from 1 to 60 cycles per minute. The duration of a complete cycle may be of any suitable length and may also occur less frequently i.e. once per hour or less. A complete cycle may also be any appropriate period, for example less than one second.

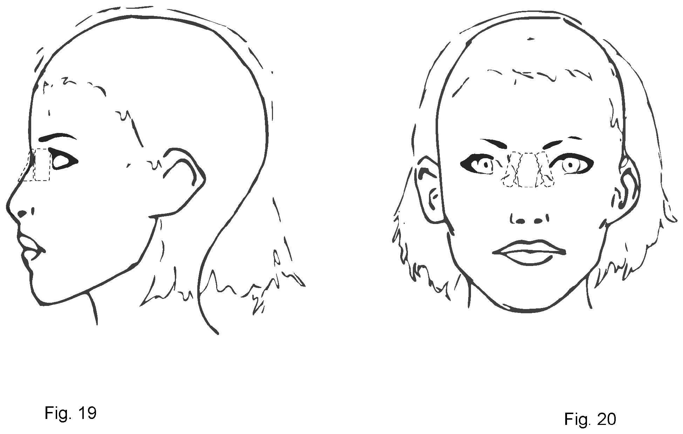

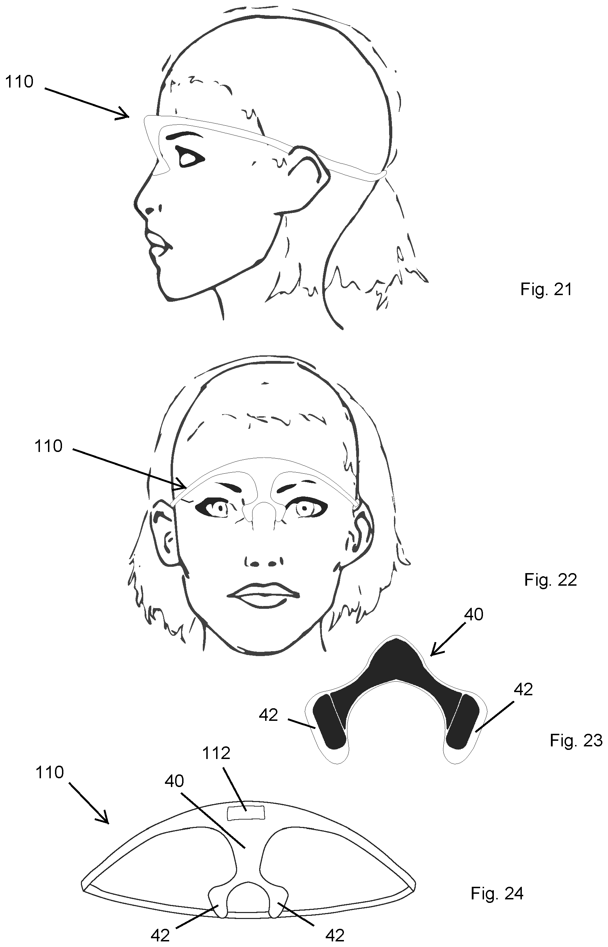

Figures 19 and 20 illustrate a target area about the nose, while Figures 21 to 24 illustrate an alternative embodiment of the tear stimulation device according to the present invention, generally indicated as 110, to apply energy to this target area. In this alternative embodiment like components have been accorded like reference numerals and unless otherwise stated perform a like function. The device 110 is provided in the form of a temple mounted frame similar to a conventional pair of glasses without lenses, the thermally active area being defined by a bridge 40 and a pair of contact pads in the form of nose pads 42 depending from the bridge 40. The hardware components of the device 110 may be provided in a housing 112 formed integrally with the frame, but could equally be located in an externally located enclosure secured to the frame, or remotely located and suitably connected to the frame, with just the energy terminal and an energy transfer interface being located on or in the frame, the bridge 40 and nose pads 42 defining the energy transfer interface. Any suitable energy transfer medium may be provide on the bridge 40 and nose pads 42 to achieve the desired energy transfer profile, for example a highly thermally conductive material to maximise thermal energy transfer from the device 110 to the target area. A suitable material is thermally conductive silicone or silicone encapsulating thermally conductive elements. The device 110 is designed to enable use with the eyes open or closed.

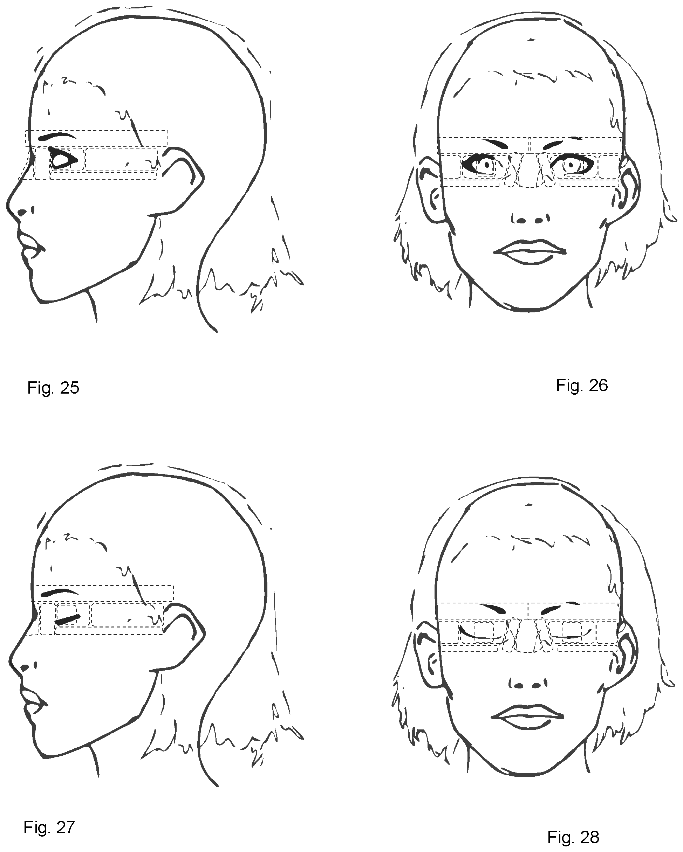

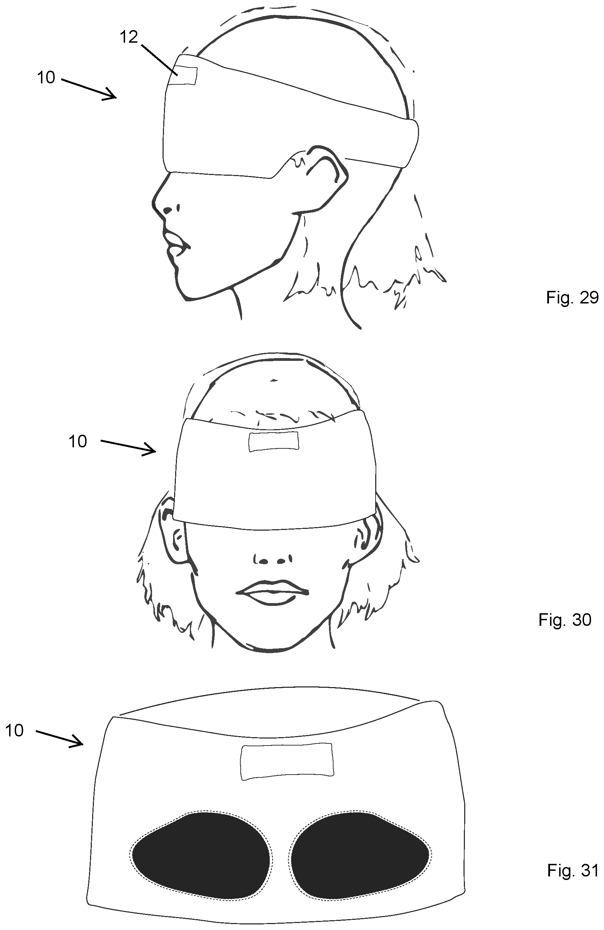

Figures 25 to 28 illustrate further target areas including the eyelids, when the eyelids are opened or closed as the thermoreceptors in the eyelids may be activated when the eyelid is open and also when closed as shown in Figures 27 and 28. Figures 29 to 31 illustrate an alternative arrangement of the headband form factor device 10 which is shaped and dimensioned to occlude the eyes when worn, and is thus intended to be worn with the eyes fully closed, for example but not exclusively during periods of sleep. Figure 31 highlights the thermally active area of the device 10, which is provided as two discrete sections shaped and dimensioned to overlie the eyes in order to apply energy to the eyelids, effectively defining contact pads for the eyes. The thermally active area of the device 10 could of course be provided with additional energy delivery zones. The housing 12 contains the hardware components of the device 10 other that the energy terminal and energy transfer interface which define the thermally active area.

The device 10 may be controlled to affect cooling to between 0 °C and 43 °C. For tear secretion the preferred temperature drop range is from 40°C to 0°C over a period of 16.5 seconds. For meibum maintenance, the preferred temperature range is 38.5 °C to 43 °C but higher temperatures may prove to be beneficial. For other skin oil glands in locations other than the eyelid, the preferred temperature range is similar to the meibum. The amount of temperature drop may vary (to avoid the thermoreceptors becoming used to the stimulus and therefore not responding). The rate of temperature drop will be from 0.01°C/s to 43 °C/s, more preferably between 3°C/s and 25°C/s, and most preferably between 5°C/s and 20°C/s. The rate of temperature drop may vary (to avoid the thermoreceptors becoming used to the stimulus and not responding). This variance may be within a single cooling phase and/or between separate cooling phases within a cycle. The duration of a cycle may vary in length, for example from 1 to 60 times per minute. The duration of a complete cycle may also occur less frequently i.e. once per hour or less. A complete cycle may also be less than one second.

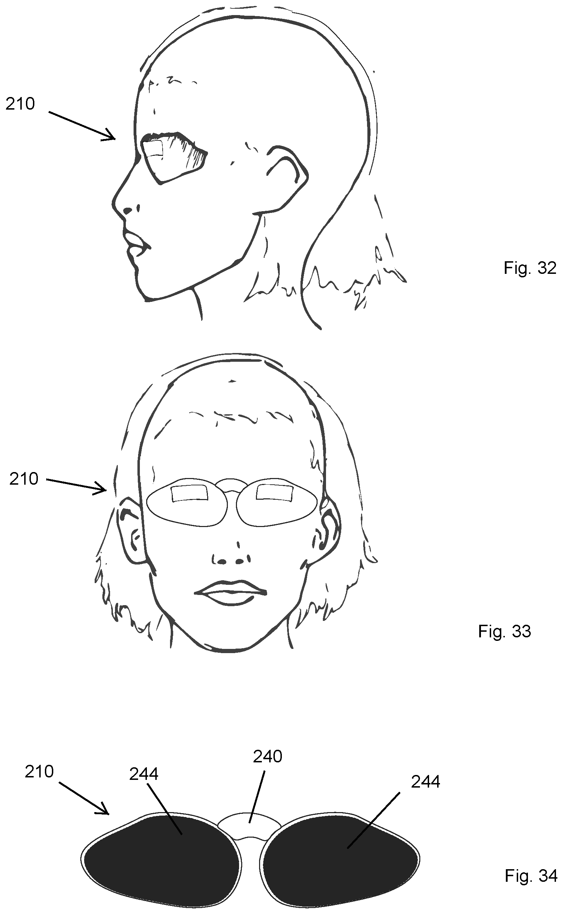

Figures 32 to 34 illustrate a further alternative embodiment of the tear stimulation device according to the present invention, generally indicated as 210. In this alternative embodiment like components have been accorded like reference numerals and unless otherwise stated perform a like function. The device 210 is provided in the form of nose mounted pair of contact pads 244 joined by a bridge 240. The hardware components of the device 210 may be provided within the bridge 240 and/or pads 244, but could equally be located in an externally located enclosure or remotely located and suitably connected to pads 244, with just the energy terminal and an energy transfer interface being located in the eye pads 244 defining the energy transfer interface. Any suitable energy transfer medium may be provide in the eye pads 244 to achieve the desired energy transfer profile, for example a highly thermally conductive gel which will facilitate thermal energy transfer and allow the pads 244 to conform to the eyelids and surrounding skin. The device 210 is intended for use with the eyes closed. However the pads 244 may be transparent and could therefore be used with the eyes opened to permit observation through the pads 244. The pads 244 could be formed of a transparent outer barrier or envelope containing a transparent energy transfer medium such as a transparent gel or the like. It will be understood that such an arrangement could be applied to other embodiments described herein.

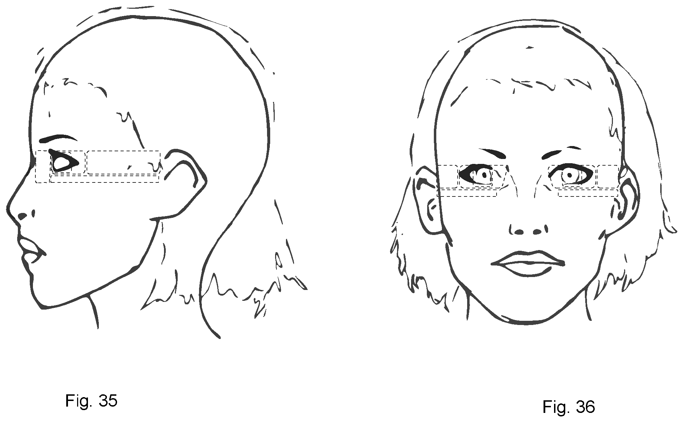

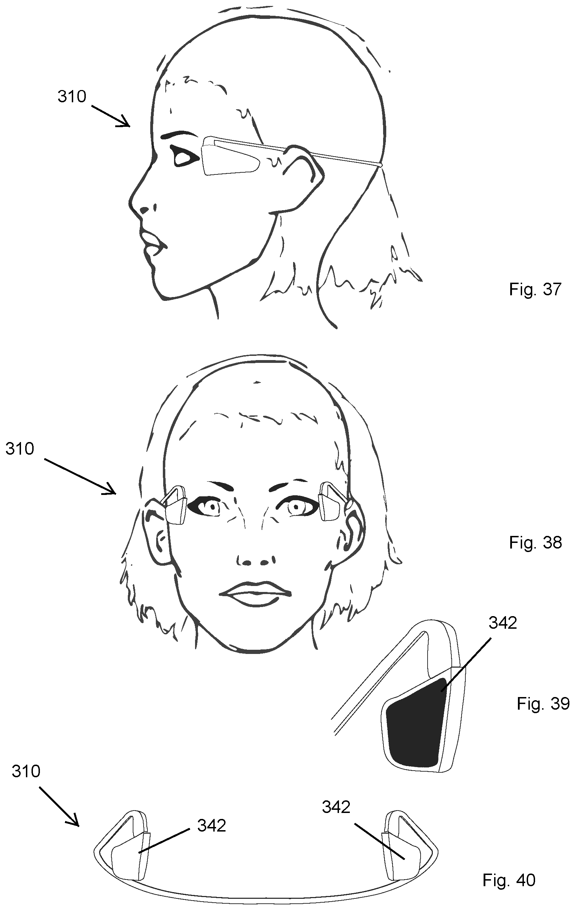

Figures 35 and 36 illustrate further target areas between the eye and temple on each side of the head. Figures 37 to 40 illustrate an alternative embodiment of the tear stimulation device according to the present invention, generally indicated as 310, adapted to deliver energy to this target area. In this alternative embodiment like components have been accorded like reference numerals and unless otherwise stated perform a like function. The device 310 is provided in the form of frame extending around the side and rear of the head, the thermally active area being defined by a pair of contact pads in the form of temple pads 342.

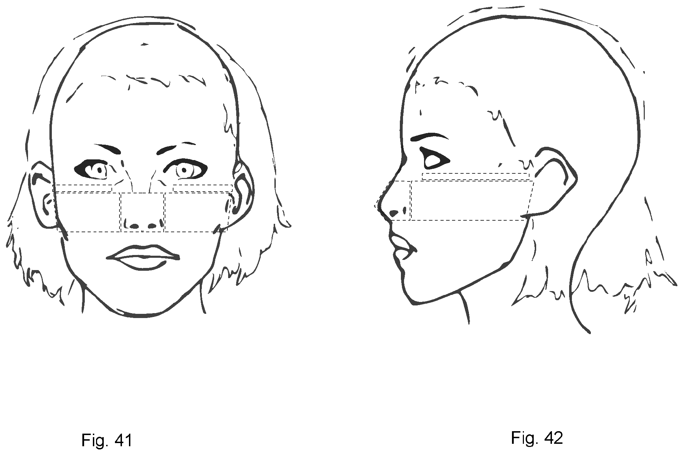

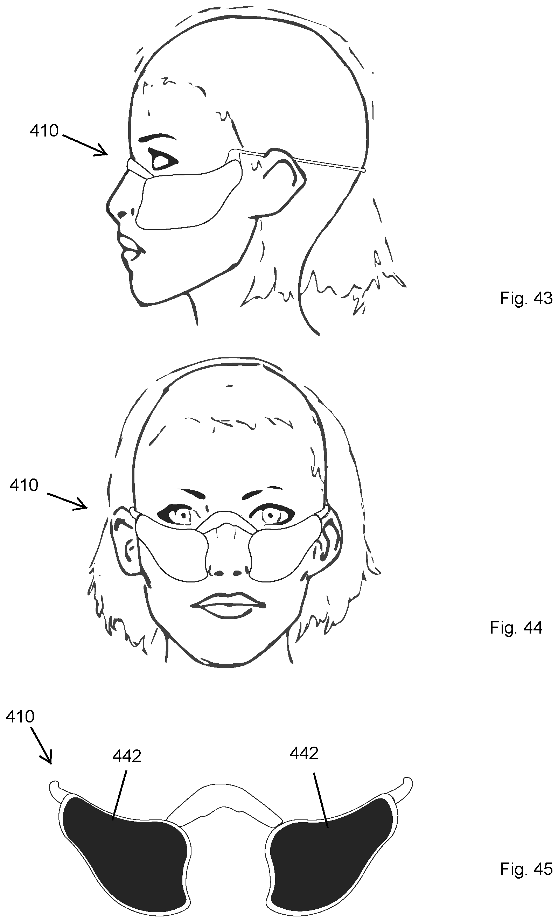

Figures 41 and 42 illustrate further target areas about the cheek. Figures 43 to 45 illustrate an alternative embodiment of the tear stimulation device according to the present invention, generally indicated as 410. In this alternative embodiment like components have been accorded like reference numerals and unless otherwise stated perform a like function. The device 410 is provided in the form of frame extending around the side and rear of the head and across the bridge of the nose, the thermally active area being defined by a pair of contact pads in the form of cheek pads 442.

It will be appreciated that for each of the above embodiments, the description of the components of the device 10 as illustrated schematically in Figure 46 apply, in addition to the various modes of operation hereinbefore described.

The tear stimulation device 10; 110; 210; 310; 410 of the present invention thus provides an effective means of delivering thermal energy at controlled rates to stimulate the creation of a natural tear by activating the thermos-receptors on the sensory nerves of the eye and extended orbital region as identified in Figures 3 and 4. Modulation of the energy, in particular by means of controlled rates of temperature change, results in controlled, repeated tearing, in particular during periods of sleep, establishing a naturally lubricated and nourishing environment in which the ocular surface can heal.

## Claims

1. A tear stimulation device comprising a housing; a controller; a power supply; and one or more energy terminals arranged about the housing for the transfer of thermal energy to and from a thermo-responsive region of the face such as to trigger or increase the involuntary activation of tear production; wherein the controller is arranged to cycle the temperature of the one or more energy terminals to deliver sequential heating and cooling phases, and wherein the controller is arranged in at least one of the cooling phases to reduce the temperature of the one or more energy terminals at a rate of between 0.01°C/s and 43°C/s, more preferably between 3°C/s and 25°C/s, and most preferably between 5°C/s and 20°C/s.

2. The tear stimulation device of claim 1 in which the controller is arranged to maintain a fixed temperature at the one or more energy terminals during one or more of the heating and/or cooling phases for a period of between 1 and 6000 seconds, more preferably between 1 and 120 seconds, and most preferably between 1 and 60 seconds.

3. The tear stimulation device of claim 1 or 2 in which the controller is arranged to set a temperature at the one or more energy terminals of between 0°C and 48°C, more preferably between 5°C and 40°C, and most preferably between 10°C and 35°C.

4. The tear stimulation device of any preceding claim in which the controller is arranged to modulate the cycle frequency and/or intensity of the thermal energy.

5. The tear stimulation device of any preceding claim in which the controller is arranged to effect a pulsed temperature change of the one or more energy terminals during at least a part of at least one cooling phase.

6. The tear stimulation device of any preceding claim in which the controller is arranged to vary the rate of temperature change during the at least one cooling phase and/or between different cooling phases.

7. The tear stimulation device of any preceding claim in which the controller is arranged to cycle the thermal energy between sequential heating and cooling phases at a frequency of two or more phases per hour, more preferably two or more phases per 10 minutes, and most preferably two or more phases per 3 minutes.

8. The tear stimulation device of any preceding claim in which the one or more energy terminals comprise a thermoelectric cooler.

9. The tear stimulation device of any preceding claim in which the one or more energy terminals are positioned to apply the thermal energy to an area on or adjacent one or more lacrimal glands or the supraorbital foramen.

10. The tear stimulation device of any preceding claim comprising one or more temperature sensors positioned at or adjacent the one or more energy terminals.

11. The tear stimulation device of any preceding claim in which the housing comprises a support operable to releasably secure the device to a user or user worn apparel.

12. The tear stimulation device of any preceding claim in which the support comprises a headband.

13. The tear stimulation device of any preceding claim in which the one or more energy terminals comprise one or more energy transfer interfaces operable to delivery thermal energy to the thermos-responsive region.
